(19) Europäisches Patentamt
European Patent Office
Office européen des brevets

(11) EP 3 166 583 B1

(12) # FASCICULE DE BREVET EUROPEEN

(45) Date de publication et mention de la délivrance du brevet:
**02.05.2018 Bulletin 2018/18**

(21) Numéro de dépôt: **15753103.9**

(22) Date de dépôt: **10.07.2015**

(51) Int Cl.:
***A61K 8/362*** *(2006.01)* ***A61Q 15/00*** *(2006.01)*
***A61K 8/41*** *(2006.01)* ***A61K 8/44*** *(2006.01)*
***A61K 8/49*** *(2006.01)*

(86) Numéro de dépôt international:
**PCT/FR2015/051917**

(87) Numéro de publication internationale:
**WO 2016/005707 (14.01.2016 Gazette 2016/02)**

(54) **UTILISATION COSMETIQUE DE L'ACIDE SPICULISPORIQUE COMME ACTIF DEODORANT**
KOSMETISCHE VERWENDUNG VON SPICULISPORSÄURE ALS DEODORANTWIRKSTOFF
COSMETIC USE OF SPICULISPORIC ACID AS A DEODORANT ACTIVE AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.07.2014 FR 1456648**

(43) Date de publication de la demande:
**17.05.2017 Bulletin 2017/20**

(73) Titulaire: **L'OREAL**
**75008 Paris (FR)**

(72) Inventeur: **SIRICHANDRA, Caroline**
**94340 Joinville le Pont (FR)**

(74) Mandataire: **Hugodot, Yannick**
**L'Oréal**
**Service DIPI**
**9 Rue Pierre Dreyfus**
**92110 Clichy (FR)**

(56) Documents cités:
**EP-A1- 2 055 314**

- **RAHMAN P K S M ET AL: "Production, Characterisation and Applications of Biosurfactants-Review", BIOTECHNOLOGY, ASIAN NETWORK FOR SCIENTIFIC INFORMATION, PK, vol. 7, no. 2, 31 décembre 2008 (2008-12-31), pages 360-370, XP002614719, ISSN: 1682-296X, DOI: 10.3923/BIOTECH.2008.360.370**
- **ISHIGAMI Y ET AL: "Surface active properties of biosoap from spiculisporic acid", JOURNAL OF COLLOID AND INTERFACE SCIENCE, ACADEMIC PRESS, NEW YORK, NY, US, vol. 94, no. 1, 1 juillet 1983 (1983-07-01), pages 131-139, XP024187869, ISSN: 0021-9797, DOI: 10.1016/0021-9797(83)90242-4 [extrait le 1983-07-01]**
- **DATABASE WPI Week 200763 Thomson Scientific, London, GB; AN 2007-671942 XP002739286, & KR 2006 0111183 A (DONGWOO FINE CHEM CO LTD) 26 octobre 2006 (2006-10-26)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 166 583 B1

## Description

La présente invention concerne l'utilisation cosmétique comme actif déodorant de l'acide spiculisporique et/ou ses sels.

La présente invention concerne l'utilisation cosmétique comme actif déodorant de l'acide spiculisporique, l'acide spiculisporique étant sous forme libre, partiellement neutralisé ou totalement neutralisé par au moins une base minérale et/ou une base organique.et plus particulièrement dans une composition comprenant un milieu physiologiquement acceptable.

Elle concerne également un procédé cosmétique pour traiter les odeurs corporelles humaines, en particulier des aisselles ou des pieds, consistant à appliquer sur les matières kératiniques humaines au moins d'un acide spiculisporique tel que défini précédemment ou une composition le contenant dans un milieu physiologiquement acceptable.

L'acide spiculisporique, également connu sous le nom du 4,5-dicarboxy-4-pentadécanolide, présente la formule suivante :

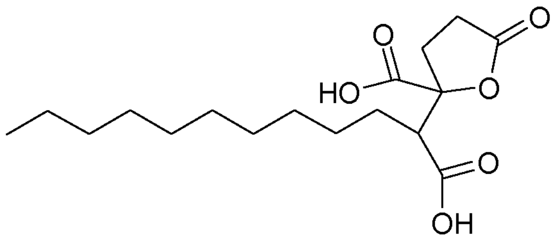

Il est connu de l'état de la technique qu'à température ambiante l'acide spiculisporique (acide-S) est insoluble dans l'eau et les corps gras mais est soluble dans l'éthanol. A température ambiante, l'acide spiculisporique peut être solubilisé dans l'eau par salification. Il a été mis en évidence la possibilité de former trois sels ; les sels de sodium ont été caractérisés :

- S-1Na, le sel mono-sodique correspondant au produit de neutralisation du groupement carboxylique lié au carbone en position C4 de l'acide-S ;
- S-2Na, le sel di-sodique correspondant au produit de neutralisation des groupements carboxyliques liés aux carbones aux positions C4 et C5 de l'acide-S ;
- S-3Na, le sel tri-sodique correspondant à la saponification de la fonction lactone de S-2Na.

Il est notamment connu que l'acide spiculisporique a des propriétés tensioactives.

On sait par ailleurs que les mauvaises odeurs de la transpiration sont en particulier liées à la présence de micro-organismes, et plus particulièrement le *Corynebacterium xerosis.* En effet, la sueur est en elle-même peut odorante lorsqu'elle est secrétée. C'est la dégradation par les bactéries *via* des réactions enzymatiques qui produit des composés malodorants. Les actifs déodorants ont précisément pour fonction de diminuer ou d'empêcher la formation des mauvaises odeurs.

Les différents systèmes proposés jusqu'à présent peuvent être regroupés en grandes familles. Parmi celles-ci, il y a les substances anti-bactériennes qui détruisent la flore bactérienne résidente. La plus employée est le Triclosan. Il y a aussi les substances qui diminuent la croissance des bactéries. Parmi ces substances, on peut citer les chélatants de métaux de transition comme l'acide éthylène diamine tétra acétique (EDTA) ou l'acide diéthylènetriamine pentaacétique (DTPA).

Cependant ces différents traitements appliqués sur la peau des aisselles ont tendance à provoquer des altérations de la peau.

Il subsiste donc le besoin de trouver de nouveaux actifs déodorants qui soient efficaces.

Dans un mode particulier il subsiste également le besoin de trouver de nouveaux actifs déodorants qui soient efficaces, et qui ne présentant pas ces inconvénients.

Les inventeurs ont mis en évidence une activité antibactérienne vis-à-vis des germes responsables des mauvaises odeurs corporelles notamment *Corynebacterium xerosis* de l'acide spiculisporique, notamment de l'acide spiculisporique neutralisé par une base organique, en particulier par la L-arginine dans un ratio molaire de 1,1, de sorte qu'il peut trouver une utilité dans une composition, de préférence cosmétique, déodorante et/ou anti-transpirante.

La présente invention concerne l'utilisation cosmétique comme actif déodorant de l'acide spiculisporique sous forme libre, partiellement neutralisé ou totalement neutralisé par au moins une base minérale et/ou une base organique, en particulier de l'acide spiculisporique partiellement ou totalement neutralisé par au moins une base organique, et plus particulièrement dans une composition comprenant un milieu physiologiquement acceptable.

Elle concerne également un procédé cosmétique pour traiter les odeurs corporelles humaines, en particulier des aisselles ou des pieds, consistant à appliquer sur les matières kératiniques humaines au moins un acide spiculisporique tel que défini précédemment ou une composition le contenant dans un milieu physiologiquement acceptable.

La présente invention concerne également une composition comprenant dans un milieu physiologiquement acceptable,

a) au moins un acide spiculisporique sous forme libre, partiellement neutralisé ou totalement neutralisé par au moins une base minérale et/ou une base organique et

b) au moins un actif déodorant additionnel et/ou au moins un actif antitranspirant.

Elle concerne aussi en particulier une composition conditionnée

(i) sous forme pressurisée dans un dispositif aérosol ou dans un flacon pompe ;

(ii) dans un dispositif muni d'une paroi ajourée notamment une grille ;

(iii) dans un dispositif muni d'un applicateur à bille (« roll-on) ;

(iv) sous forme de bâtonnet (stick); ou

(v) sous forme de poudre libre ou compactée,

caractérisée par le fait qu'elle contient dans un milieu physiologiquement acceptable un acide spiculisporique tel que défini précédemment.

Dans le cadre de la présente invention on appelle « actif déodorant », toute substance capable de masquer, absorber, améliorer et/ou réduire l'odeur désagréable résultant de la décomposition de la sueur humaine par des bactéries Par « actif anti-transpirant », on entend un sel qui, à lui seul, a pour effet de diminuer le flux sudoral, de diminuer la sensation sur la peau d'humidité liée à la sueur humaine ou de masquer la sueur humaine.

Au sens de la présente invention, on entend désigner par « milieu physiologiquement acceptable », un milieu convenant à l'administration d'une composition par voie topique.

Un milieu physiologiquement acceptable est généralement sans odeur, ou aspect désagréable, et qui est parfaitement compatible avec la voie d'administration topique.

Dans le cas présent où la composition est destinée à être administrée par voie topique, c'est à dire par application en surface de la matière kératinique considérée, un tel milieu est en particulier considéré comme physiologiquement acceptable lorsqu'il ne génère pas de picotement, tiraillement ou rougeur inacceptable pour l'utilisateur.

## ACIDE SPICULISPORIQUE

L'acide spiculisporique, également connu sous le nom du 4,5-dicarboxy-4-pentadécanolide, présente la formule suivante :

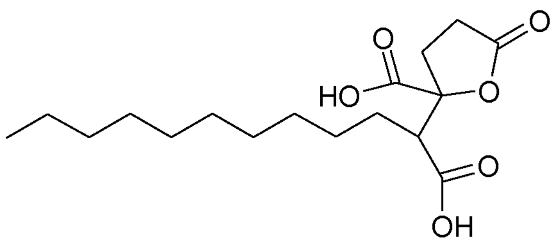

Dans le cadre de la présente invention, l'acide spiculisporique peut être sous forme libre, partiellement neutralisé ou totalement neutralisé par au moins une base minérale et/ou une base organique, et plus particulièrement dans une composition comprenant un milieu physiologiquement acceptable.

Dans un mode particulier selon l'invention, l'acide spiculisporique peut être sous forme libre, partiellement neutralisé ou totalement neutralisé par au moins une base minérale et/ou une base organique dans une composition comprenant un milieu physiologiquement acceptable, et comprenant en outre au moins un tensioactif.

Dans le cadre de l'invention, et sauf mention contraire, le ratio R1 correspond au ratio du nombre de moles de base sur le nombre de moles d'acide spiculisporique. Il s'agit donc d'un ratio molaire. On peut par exemple citer un ratio molaire R1 strictement supérieur à 1.0, et de préférence inférieur ou égal à 2.50. Ainsi, un ratio molaire R1 strictement supérieur à 1.0 correspond à un nombre de moles de base strictement supérieur au nombre de moles de l'acide spiculisporique.

Selon un mode de réalisation, le ratio R1 est strictement supérieur à 1.0. De préférence, le ratio R1 est compris

entre 1.0 et 2.50. Plus particulièrement le ratio R1 est supérieur ou égal à 1.10 et inférieur ou égal à 2.0.

En particulier, le ratio R1 est égal à 1,10.

Selon un mode de réalisation, le ratio nommé R2, de la masse d'acide spiculisporique sur la masse de tensioactif(s), différent(s) du S-acide, est inférieur ou égal à 12.50.

Selon un mode de réalisation, le ratio R2 est compris entre 0,1 et 12,5. En particulier, le ratio R2 est compris entre 1.0 et 12.50. De préférence, le ratio R2 est compris entre 1.0 et 5.0.

Dans le cadre de l'invention, et sauf mention contraire, le ratio R2 correspond au ratio de la masse d'acide spiculisporique sur la masse de tensioactif(s). Il s'agit donc d'un ratio massique.

A titre d'exemple, l'acide spiculisporique est disponible sous la dénomination commerciale SPICULISPORIC ACID® de la société IWATA CHEMICAL.

Selon l'invention, la teneur en acide spiculisporique peut aller de 0,1% à 15% en masse par rapport à la masse totale de ladite composition.

Selon un mode de réalisation préféré, la teneur en acide spiculisporique est comprise entre 0,1% et 15%, de préférence entre 0,5% et 10%, et préférentiellement entre 1% et 10% en masse de matière active par rapport à la masse totale de la composition.

## COMPOSITION

### Tensioactifs

Dans un mode de réalisation particulier de l'invention, l'acide spiculisporique est compris dans une composition comprenant un milieu physiologiquement acceptable, qui comprend en outre au moins un tensioactif.

Le ou les tensioactifs selon l'invention sont choisis parmi les tensioactifs sulfatés et/ou sulfonatés, et les tensioactifs non sulfatés et non sulfonatés.

Dans un mode de réalisation particulier l'acide spiculisporique est compris dans une composition comprenant un milieu physiologiquement acceptable, qui comprend en outre au moins une base organique et au moins un tensioactif sulfaté et/ou sulfonaté.

Selon un mode de réalisation, le tensioactif sulfaté et/ou sulfonaté est choisi dans le groupe constitué des tensioactifs anioniques sulfatés et/ou sulfonatés.

Selon un mode de réalisation, le tensioactif sulfaté et/ou sulfonaté est un tensioactif anionique sulfaté, un tensioactif anionique sulfonaté ou un mélange de ces tensioactifs.

Dans le cadre de l'invention, et sauf mention contraire, le terme "tensioactif anionique sulfaté" désigne un tensioactif comprenant un groupement sulfate, à savoir un tensioactif anionique comprenant un groupement $-OSO_3^-$ ou $-OSO_3H$. Selon l'invention, le terme "tensioactif anionique sulfonaté" désigne un tensioactif comprenant un groupement sulfonate, à savoir un tensioactif anionique comprenant un groupement $-SO_3^-$ ou $-SO_3H$.

Selon un mode de réalisation, les tensioactifs anioniques sulfatés ou sulfonatés selon l'invention sont choisis dans le groupe constitué des alkylsulfates, des alkyléthersulfates, des alkylamidoéthersulfates, des alkylarylpolyéthersulfates, des monoglycéride-sulfates, des sulfonates, des iséthionates, des taurates, des sulfosuccinates, des alkyl sulfoacétates, ainsi que des formes salifiées correspondantes, et de leurs mélanges.

Ces composés peuvent être oxyéthylénés et comportent alors de préférence de 1 à 50 motifs oxyde d'éthylène, mieux de 1 à 10 motifs oxyde d'éthylène.

Lorsque le tensioactif anionique est sous forme de sel, il peut être choisi parmi les sels de métaux alcalins tels que le sel de sodium ou de potassium, les sels d'ammonium, les sels d'amines et en particulier d'aminoalcools ou les sels de métaux alcalino-terreux tels que le sel de magnésium.

Comme exemple de sels d'aminoalcools, on peut citer les sels de mono-, di- et triéthanolamine, les sels de mono-, di- ou tri-isopropanolamine, les sels de 2-amino 2-méthyl 1-propanol, 2-amino 2-méthyl 1,3-propanediol et tris(hydroxyméthyl)amino méthane.

On utilise de préférence les sels de métaux alcalins ou alcalinoterreux, et en particulier les sels de sodium ou de magnésium.

Parmi les tensioactifs anioniques sulfonatés, on peut en particulier citer les alkylsulfonates, les alkylamidesulfonates, les alkylarylsulfonates, les alpha-oléfine-sulfonates, les paraffine-sulfonates et leurs mélanges.

Parmi les iséthionates, on peut en particulier mentionner les acyliséthionates. Parmi les taurates, on peut en particulier mentionner les N-acyltaurates, les N-méthyltaurates et les formes acides correspondantes.

Pour tous les composés susmentionnés, les groupes alkyle et acyle comportent de préférence de 6 à 30 atomes de carbone, mieux de 12 à 24, voire de 16 à 22 atomes de carbone, et les groupes aryles sont de préférence un groupe phényle ou benzyle.

Selon un mode de réalisation, le tensioactif sulfaté et/ou sulfonaté selon l'invention est choisi dans le groupe constitué des alkylsulfates, des alkyléthersulfates, des sulfonates, des iséthionates, des taurates, des sulfosuccinates,

des alkyl sulfoacétates et de leurs mélanges.

Selon l'invention, les tensioactifs anioniques sulfatés ou sulfonatés peuvent également être choisis parmi les alkylsulfosuccinates, les alkyléthersulfosuccinates, les alkylamide-sulfosuccinates, et les formes acides correspondantes, les groupes alkyles de ces composés comportant de 6 à 30 atomes de carbone, mieux de 12 à 24, voire de 16 à 22 atomes de carbone.

A titre d'exemples de tensioactif anionique sulfaté et/ou sulfonaté, on peut citer plus particulièrement le laurylsulfate de sodium, le laurethsulfate de sodium, le laurylméthyl iséthionate de sodium, le lauryl sulfate de triéthanolamine, le lauryl sulfate d'ammonium, le cétostéaryl sulfate de sodium, et leurs mélanges.

Comme sulfonates, on peut citer par exemple les alpha-oléfines sulfonates comme l'alpha-oléfine sulfonate de sodium (C14-16) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.

Comme alkyl sulfoacétates, on peut citer le laurylsulfoacétate comme par exemple celui qui est commercialisé en mélange avec le méthyl-2-sulfolaurate de sodium et le sulfolaurate-2-de disodium sous la référence STEPAN MILD PCL par la société Stepan. On peut aussi citer le sel de sodium de lauryl sulfoacétate sous le nom INCI SODIUM LAURYL SULFOACETATE et commercialisé sous le nom LATHANOL LAL® par la société STEPAN.

Comme iséthionates, on peut citer les acyliséthionates comme le cocoyl-iséthionate de sodium, tel que le produit commercialisé sous la dénomination JORDAPON CI P® par la société Jordan, ainsi que le lauroyl méthyl iséthionate de sodium (par exemple ISELUX LQ-CLR-SB de INNOSPEC).

Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le sel di-sodique de mono-sulfosuccinate d'alcool laurique commercialisé sous la dénomination REWOPOL SB F12P® par la société Witco, le mono-sulfosuccinate de mono-éthanolamide ricinoléique commercialisé sous la dénomination REWODERM S 1333® par la société Witco. On peut utiliser aussi les sulfosuccinates de polydiméthylsiloxane tels que le disodium PEG-12 diméthicone sulfosuccinate commercialisé sous la dénomination MACKANATE-DC30 par la société Mac Intyre.

Comme alkyl éther sulfates, on peut citer par exemple le lauryl éther sulfate de sodium (nom CTFA : sodium laureth sulfate) comme celui commercialisé sous les dénominations TEXAPON® N40 et TEXAPON® AOS 225 UP par la société Cognis, le lauryl éther sulfate d'ammonium (nom CTFA : ammonium laureth sulfate) comme celui commercialisé sous la dénomination STANDAPOL® EA-2 par la société Cognis, ou encore l'alkyl (C12-C14) éther (9 OE) sulfate d'ammonium commercialisé sous la dénomination RHODAPEX AB/20® par la société Rhodia Chimie.

Comme alkyl sulfates, on peut citer par exemple le lauryl sulfate de sodium (nom CTFA : sodium lauryl sulfate) tel que le produit commercialisé par la société Tensachem sous la dénomination TENSOPOL USP94, le lauryl sulfate de triéthanolamine (nom CTFA : TEA-lauryl sulfate) tel que le produit commercialisé par la société Huntsman sous la dénomination EMPICOL® TL40 FL ou celui commercialisé par la société Cognis sur la dénomination TEXAPON® T42, produits qui sont à 40% en solution aqueuse. On peut aussi citer le lauryl sulfate d'ammonium (nom CFTA : Ammonium lauryl sulfate) tel que le produit commercialisé par la société Huntsman sous la dénomination EMPICOL® AL 30FL qui est à 30% en solution aqueuse.

Selon un mode de réalisation, le tensioactif sulfaté et/ou sulfonaté est choisi dans le groupe constitué des alkyl sulfates, des alkyléthersulfates, des iséthionates et de leurs mélanges.

En particulier, le tensioactif sulfaté et/ou sulfonaté est choisi dans le groupe constitué des alkyl($C_6$-$C_{24}$) sulfates, des alkyl($C_6$-$C_{24}$) éther sulfates comprenant de 1 à 20, de préférence de 2 à 10, motifs oxyde d'éthylène, des iséthionates, et de leurs mélanges, notamment sous forme de sels de métaux alcalins, d'ammonium, d'aminoalcools, ou de métaux alcalino-terreux, ou un mélange de ces composés.

En particulier, on préfère utiliser les alkyl($C_{12-20}$) sulfates, les alkyl($C_{12-20}$) éther sulfates comprenant de 1 à

20, de préférence de 2 à 10, motifs oxyde d'éthylène, les iséthionates, notamment sous forme de sels de métaux alcalins, d'ammonium, d'aminoalcools, et de métaux alcalino-terreux, ou un mélange de ces composés. Selon un mode de réalisation, le tensioactif sulfaté et/ou sulfonaté est choisi dans le groupe constitué du lauryl sulfate de sodium, du lauryl éther sulfate de sodium comprenant 2 à 10 motifs oxyde d'éthylène tel que les laureth sulfates commercialisés sous les dénominations TEXAPON AOS 225 UP de COGNIS ou IFRAPON LOS 70 RO 16 de ECOGREEN OLEOCHEMICALS, du lauryl méthyl iséthionate de sodium et de leurs mélanges.

Les compositions cosmétiques de l'invention peuvent comprendre un unique tensioactif tel que défini ci-dessus, ou un mélange de tensioactifs tels que définis ci-dessus.

Selon l'invention, le tensioactif non sulfaté et non sulfonaté peut être un tensioactif non sulfaté et non sulfonaté choisi dans le groupe constitué des tensioactifs amphotères, des tensioactifs anioniques, des tensioactifs non ioniques, des tensioactifs cationiques, et leurs mélanges.

Ce tensioactif n'est pas un tensioactif anionique comportant un groupement sulfate ou sulfonate.

Dans le cadre de l'invention, et sauf mention contraire, on entend par tensioactif anionique comprenant un groupement sulfate, un tensioactif anionique comprenant un groupement -OSO3- ou -OSO3H (tensioactif sulfaté) et par tensioactif anionique comprenant un groupement sulfonate (tensioactif sulfonaté), un tensioactif anionique comprenant un groupement -SO3- ou -SO3H.

Dans le cadre de l'invention, le « tensioactif non sulfaté et non sulfonaté » peut être un tensioactif non sulfaté (ne comprenant pas de groupement sulfate), un tensioactif non sulfonaté (ne comprenant pas de groupe sulfonate) ou un tensioactif non sulfaté et non sulfonaté (ne comprenant ni groupe sulfate ni groupe sulfonate).

Selon l'invention, le tensioactif peut être un tensioactif amphotère.

Les tensioactifs amphotères (ce terme incluant les tensioactifs amphotères et zwitterioniques) peuvent être choisis par exemple parmi les bétaïnes, les N-alkylamidobétaïnes et leurs dérivés, les dérivés de la glycine, les sultaïnes, les alkyl polyaminocarboxylates, les alkylamphoacétates, les lécithines et leurs mélanges.

Comme bétaïnes, on peut citer notamment les alkylbétaïnes comme par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica.

Parmi les N-alkylamidobétaïnes et leurs dérivés, on peut citer par exemple la cocamidopropyl bétaïne commercialisée sous la dénomination LEBON 2000 HG® par la société Sanyo, sous la dénomination EMPIGEN BB® par la société Albright & Wilson, sous les dénominations Tego Betain F 50 et CK D par la société EVONIK GOLDSCHMIDT, ou encore celles commercialisées en mélange avec du glycéryl lauratec comme les références commerciales Tego Betain HS ou Antil HS 60 d'EVONIK GOLDSCHMIDT, la lauramidopropyl bétaïne commercialisée sous la dénomination REWOTERIC AMB12P® par la société Witco.

Comme sultaïnes, on peut citer le cocoyl-amidopropylhydroxy-sulfobetaïne commercialisé sous la dénomination CROSULTAINE C-50® par la société Croda. Comme alkyl polyaminocarboxylates (APAC), on peut citer le cocoylpolyamino-carboxylate de sodium, commercialisé sous la dénomination AMPHOLAK 7 CX/C®, et AMPHOLAK 7 CX® par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacétate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom CTFA : sodium cocamphoacétate.

Parmi les lécithines, on peut mentionner les phospholipides et les lysophospholipides. En particulier, on peut citer les lécithines commercialisées par NIKKOL group (Lecinol Series) ou encore les lécithines commercialisées par CARGIL/LUCAS MEYER (la famille des EMULMETIK des EMULFLUIDEd). Selon un mode de réalisation, le tensioactif amphotère est choisi dans le groupe constitué des bétaïnes, les N-alkylamidobétaïnes et leurs dérivés, les dérivés de la glycine, les sultaïnes, les alkyl polyaminocarboxylates, les alkylamphoacétates, les lécithines et leurs mélanges.

Selon un mode de réalisation, le tensioactif amphotère est choisi dans le groupe constitué des alkylbétaïnes, des alkylamidopropylbétaïnes, des alkylamphoacétates, et de leurs mélanges.

Selon un mode de réalisation, le tensioactif est un tensioactif amphotère choisi dans le groupe constitué du cocoyl bétaïne, du cocamidopropylbétaïne, du cocoamphodiacétate disodique, du cocoamphoacétate de sodium, et de leurs mélanges.

Selon un mode de réalisation, le tensioactif est le cocamidopropylbétaïne ou le cocoamphodiacétate disodique.

Selon l'invention, le tensioactif peut être un tensioactif anionique non sulfaté et non sulfonaté.

Les tensioactifs anioniques peuvent être choisis notamment parmi les dérivés anioniques de protéines d'origine

végétale, les aminoacides et les dérivés des aminoacides, les phosphates et alkylphosphates, les polypeptides, les dérivés anioniques d'alkyl polyglucoside, les savons (sels d'acides gras), les dérivés de l'huile de soja, les dérivés d'acide lactique, leurs sels et leurs mélanges, les acides glycyrrhiziques ou les lipopeptides (comme le biosurfactant Surfactine lipopeptide d'origine microbienne).

Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale, et le groupement hydrophobe peut être notamment une chaîne grasse, par exemple une chaîne alkyle comportant de 10 à 22 atomes de carbone.

Comme dérivés anioniques de protéines d'origine végétale, utilisables dans la composition selon l'invention, on peut plus particulièrement citer les hydrolysats de protéines de blé, de soja, d'avoine ou de soie, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium. On peut citer par exemple les sels de sodium, de potassium et/ou d'ammonium des hydrolysats de protéine de soie modifiée par l'acide laurique, tels que le produit commercialisé sous la dénomination KAWA SILK par la société Kawaken ; les sels de sodium, de potassium et/ou d'ammonium des hydrolysats de protéine de blé modifiée par l'acide laurique, tels que le sel de potassium commercialisé sous la dénomination AMINOFOAM W OR par la société Croda (nom CTFA : Potassium lauroyl wheat aminoacids) et le sel de sodium commercialisé sous la dénomination PROTEOL LW 30 par la société Seppic (nom CTFA : sodium lauroyl wheat aminoacids) ; les sels de sodium, de potassium et/ou d'ammonium des hydrolysats de protéine d'avoine comportant une chaîne alkyle ayant de 10 à 22 atomes de carbone, et plus spécialement les sels de sodium, de potassium et/ou d'ammonium des hydrolysats de protéine d'avoine modifiée par l'acide laurique, tels que le sel de sodium commercialisé sous la dénomination PROTEOL OAT (nom CTFA : Sodium lauroyl oat aminoacids), le PROTEOL SAV 50S (nom INCI : Sodium cocoyl aminoacid), le PROTEOL APL (nom INCI : sodium cocoyl apple amino acids) par la société Seppic, l'AMARANTH S (nom INCI : sodium cocoyl hydrolyzed amaranth proteins) et leurs mélanges.

Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie.

Les dérivés anioniques d'alkyl-polyglucosides peuvent être notamment des citrates, tartrates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel de sodium d'ester citrique de cocoyl polyglucoside (1,4) commercialisé sous la dénomination EUCAROL AGE-EC® par la société Cesalpinia.

Les savons peuvent être obtenus à partir d'un acide gras qui est partiellement ou totalement saponifié (neutralisé) par un agent basique. Ce sont des savons de métal alcalin ou alcalino-terreux ou de bases organiques. Comme acides gras, on peut utiliser les acides gras saturés, linéaires ou ramifiés, comportant de 8 à 30 atomes de carbone, et de préférence comportant de 8 à 22 atomes de carbone. Cet acide gras peut être en particulier choisi parmi l'acide palmitique, l'acide stéarique, l'acide myristique, l'acide laurique, et leurs mélanges.

Comme agents basiques, on peut utiliser par exemple les hydroxydes de métaux alcalins (hydroxyde de sodium et hydroxyde de potassium ou potasse), les hydroxydes de métaux alcalino-terreux (par exemple de magnésium), l'hydroxyde d'ammonium, ou encore les bases organiques comme la triéthanolamine, la N-méthylglucamine, la lysine et l'arginine.

Les savons peuvent être notamment des sels alcalins d'acide gras, l'agent basique étant un hydroxyde de métal alcalin, et de préférence l'hydroxyde de potassium ou potasse (KOH).

La quantité d'agent basique doit être suffisante pour que l'acide gras soit au moins partiellement neutralisé.

On peut citer notamment le laurate de sodium ou de potassium, le myristate de potassium, le palmitate de potassium, le stéarate de potassium, le cocoate de potassium ou encore les sels d'acide stéarique de KOH formés in situ.

Les dérivés de l'huile de soja et leurs sels sont en particulier les acides gras et sels d'acides gras dérivés de l'huile de soja (dont le nom INCI est « glycine soja oil » ou « soybean oil ») et en particulier les sels de métaux alcalins tels que Na, Li, K, de préférence Na ou K, et d'acides gras issus du soja, tels que le potassium soyate comme par exemple celui qui est commercialisé par la société Noveon.

Comme acylaminoacides, on peut citer par exemple le cocoylglycinate de sodium commercialisé par la société Ajinomoto sous la dénomination Amilite GCS-12, le cocoylglycinate de sodium commercialisé par la société Ajinomoto sous la dénomination Amilite GCK-12, le cocoyl glutamate de disodium commercialisé par la société Ajinomoto sous la dénomination Amisoft ECS-22SB, le lauroyl glutamate de sodium commercialisé par la société Ajinomoto sous la dénomination Amisoft LS11, le lauroyl sarcosinate de sodium commercialisé par la société Seppic sous la dénomination ORAMIX L 30, les sodium et disodium stearoyl glutamate commercialisés par la société Ajinomoto sous les dénomination Amisoft HS21 P et HS11 Pf et le cocoyl sarcosinate de sodium commercialisé par la société ZSCHIMMER & SCHWARZ sous la dénomination Protelan LS 9011/C. On peut également citer le sel de sodium de lauroyl aminoacides d'avoine tel que le Proteol OAT commercialisé par la société Seppic ou le composé portant le nom INCI sodium cocoyl aminoacids tel que le Proteol SAV 5OS de Seppic.

Les dérivés des aminoacides peuvent être choisis par exemple parmi les sarcosinates, et notamment les acylsarcosinates comme le lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium, commercialisé sous la dénomination NIKKOL SARCOSINATE MN® par la société Nikkol, le palmitoyl sarcosinate de sodium, commercialisé sous la dénomination NIKKOL SARCOSINATE PN ® par la société Nikkol ; les alaninates comme le N-lauroyl-N-méthylamidopropionate de sodium, commercialisé sous la dénomination SODIUM NIKKOL ALANINATE LN 30® par la société Nikkol ou commercialisé sous la dénomination ALANONE ALE®, par la société Kawaken, et le N-lauroyl N-methylalanine triéthanolamine, commercialisé sous la dénomination ALANONE ALTA ® par la société Kawaken ; les aspartates comme le mélange de N-lauroylaspartate de triéthanolamine et de N-myristoylaspartate de triéthanolamine, commercialisé sous la dénomination ASPARACK ® par la société Mitsubishi et les citrates.

On peut également citer les sels alcalins des acides acyl (C10-C22) glutamique, de préférence un sel alcalin des acides acyl (C12-C20) glutamiques, et par exemple un sel alcalin des acides acyl (C16-C18) glutamiques. Les sels alcalins, sont par exemple les sels de sodium, les sels de potassium et les sels de lithium, et de préférence les sels de sodium.

Il peut notamment s'agir d'un des sels alcalins de l'acide stéaroyl glutamique, de l'acide lauroyl glutamique, de l'acide acyl C16 glutamique, de l'acide myristoyl glutamique, de l'acide cocoyl glutamique ou de l'acide acyle de suif hydrogéné glutamique.

De préférence, il s'agit d'un tensioactif ionique choisi parmi le stéaroyl glutamate de sodium, le stéaroyl glutamate disodique, le stéaroyl glutamate de potassium, le lauroyl glutamate de sodium, le lauroyl glutamate disodique, le lauroyl glutamate de potassium, le cocoyl glutamate de sodium, l'acyle de suif hydrogéné glutamate de sodium, et leurs mélanges, et de préférence il s'agit du stéaroyl glutamate de sodium.

A titre illustratif, on peut, par exemple, citer le stéroyl glutamate de sodium commercialisé par la société AJINOMOTO sous la référence AMISOFT HS 11 PF®.

Les dérivés d'acides lactiques ou leurs sels peuvent être choisis parmi les dérivés d'acide acyl lactylique, leurs sels (lactylates) tels que le stéaroyl lactylate tel que par exemple celui commercialisé par la société Oleon NV sous le nom Radiamuls 2980 ; le sodium stéaroyl lactylate tel que proposé par exemple par la société Oleon NV sous la dénomination Radiamuls 2990, par la société Karlshamns AB sous le nom Akoline SL, par la société Uniquema sous le nom Priazul 2134 ou encore par Dr Straetmans sous le nom Dermofeel SL ; le sodium isostéaroyl lactylate tel que celui commercialisé par Uniquema sous le nom Priazul 2133 ; le sodium behenoyl lactylate par exemple commercialisé par la société Rita Corporation sous le nom Pationic SBL ; le sodium cocoyl lactylate tel que celui commercialisé par la société Rita sous le nom Pationic SCL, le sodium oleoyl lactylate, le sodium lauroyl lactylate (PATIONIC 138C de Caravan), le sodium caproyl lactylate (CAPMUL S8L-G de Abitec).

On peut également citer le mélange cocoamphoacétate de sodium, glycérine, lauryl glucoside, cocoylglutamate de sodium, lauryl glucose carboxylate de sodium, commercialisé par la société Cognis sous la référence Plantapon SF.

En particulier, le tensioactif anionique est choisi dans le groupe constitué des carboxylates gras, des sarcosinates d'alkyle, des phosphates d'alkyle, des alkylglutamates, des acylglutamates, et de leurs mélanges.

Selon un mode de réalisation, le tensioactif est un tensioactif anionique est choisi dans le groupe constitué du cocoyl glutamate disodique, du lauryl sarcosinate de sodium, et de leurs mélanges.

Selon un mode de réalisation, le tensioactif est le cocoyl glutamate disodique ou le lauryl sarcosinate de sodium.

Selon l'invention, le tensioactif peut être un tensioactif non ionique.

Les tensioactifs non ioniques peuvent être choisis par exemple parmi les alkyl polyglucosides (APG), les alkylpolypentosides, les alkylpolyxylosides, les esters de maltose, les esters de sucrose, les gommes hydrophobées, les alcools gras polyglycérolés, les esters de sucre oxyalkylénés, les esters d'acide gras et de polyéthylène glycol, les esters d'acide gras et de sorbitan, les dérivés de glucamine comme l'éthyl-2 hexyl oxy-carbonyl N-méthyl glucamine, et leurs mélanges.

Comme alkylpolyglucosides, on utilise de préférence ceux contenant un groupe alkyle comportant de 6 à 30 atomes de carbone, et de préférence de 8 à 16 atomes de carbone, et contenant un groupe hydrophile (glucoside)

comprenant de préférence 1,2 à 3 unités de saccharide. On peut citer par exemple le décylglucoside (Alkyl-C9/C11-polyglucoside (1.4)) comme le produit commercialisé sous la dénomination MYDOL 10® par la société Kao Chemicals, le produit commercialisé sous la dénomination PLANTAREN 2000 UP ® par la société Cognis, et le produit commercialisé sous la dénomination ORAMIX NS 10 ® par la société Seppic ; le caprylyl/capryl glucoside comme le produit commercialisé sous la dénomination ORAMIX CG 110 ® par la société Seppic ou PLANTACARE 810 P par la société Cognis ; le laurylglucoside comme les produits commercialisés sous les dénominations PLANTAREN 1200 N ® et PLANTACARE 1200 ® par la société Cognis ; et le coco-glucoside comme le produit commercialisé sous la dénomination PLANTACARE 818/UP ® par la société Cognis, le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic, les C12 à C20 alkyl glucosides tels que ceux commercialisés en mélange avec des alcools gras en C14 à C22 sous la référence MONTANOV L par la société Seppic.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioléate commercialisé sous la dénomination Glucamate DOE 120 par la société Amerchol.

Les esters d'acide gras et de polyéthylène glycol sont de préférence des esters d'acides gras en C16-C22 comportant de 8 à 100 unités d'oxyde éthylène.

La chaîne grasse des esters peut être notamment choisie parmi les motifs stéaryle, béhényle, arachidyle, palmityle, cetyle et leurs mélanges, tel que cétéaryle, et de préférence une chaîne stéaryle.

Le nombre d'unités d'oxyde d'éthylène peut aller de 8 à 100, de préférence de 10 à 80, et mieux de 10 à 50. Selon un mode particulier de réalisation de l'invention, ce nombre peut aller de 20 à 40.

A titre d'exemple d'ester d'acide gras et de polyéthylène glycol, on peut citer les esters d'acide stéarique comprenant respectivement 20, 30, 40, 50, 100 unités d'oxyde d'éthylène, tels que les produits commercialisés respectivement sous la dénomination Myrj 49 P (stéarate de polyéthylène glycol 20 OE ; nom CTFA : PEG-20 stearate), Myrj 51, Myrj 52 P (stéarate de polyéthylèneglycol 40 OE ; nom CTFA : PEG-40 stearate), Myrj 53, Myrj 59 P par la société CRODA.

Les esters d'acide gras en C16-C22 et de sorbitan sont en particulier des esters d'acides en C16-C22 et de sorbitan et sont formés par estérification d'au moins un acide gras comportant au moins une chaîne alkyle linéaire saturée ou insaturée, ayant respectivement de 16 à 22 atomes de carbone, avec le sorbitol. Ces esters peuvent être notamment choisis parmi les stéarates, béhénates, arachidates, palmitates, oléates de sorbitan, et leurs mélanges. On utilise de préférence des stéarates et palmitates de sorbitan, et préférentiellement les stéarates de sorbitan.

On peut citer à titre d'exemple d'ester de sorbitan utilisable dans composition selon l'invention, le monostéarate de sorbitan (nom CTFA : Sorbitan stearate) vendu par la société Croda sous la dénomination Span 60, le tristéarate de sorbitan vendu par la société Croda sous la dénomination Span 65 V, le monopalmitate de sorbitan (nom CTFA : Sorbitan palmitate) vendu par la société Croda sous la dénomination Span 40, le monoléate de sorbitan vendu par la société Croda sous la dénomination Span 80 V , le trioléate de sorbitan vendu par la société Uniquema sous la dénomination Span 85 V. de préférence, l'ester de sorbitan utilisé est le tristéarate de sorbitan.

Les esters de sucrose et d'acides gras sont de préférence choisis parmi les esters issus de la réaction de sucrose(s) (saccharose) et d'acide(s) gras comprenant de 10 à 24 atomes de carbone, de préférence de 12 à 20 atomes de carbone, mieux de 12 à 18 atomes de carbone et encore mieux de 12 à 16 atomes de carbone. Les acides gras comprenant de 10 à 24 atomes de carbone peuvent être linéaires ou ramifiés, saturés ou insaturés.

Les acides gras peuvent être choisis parmi l'acide oléique, l'acide laurique, l'acide palmitique, l'acide myristique, l'acide stéarique, l'acide linoléique, l'acide caprique, ou leurs mélanges

Selon un mode de réalisation, l'ester de sucrose et d'acide gras est choisi parmi les esters issus de la réaction de sucrose et d'acide gras comprenant de 12 à 18 atomes de carbone, de préférence de 12 à 16 atomes de carbone tels que l'acide laurique et/ou l'acide palmitique comme par exemple le sucrose laurate, le sucrose palmitate ou un mélange.

Les esters de sucrose et d'acides gras peuvent être choisis parmi les mono-, di-, tri- et tétra-esters, les polyesters et leurs mélanges. On utilise de préférence des esters à faible degré d'estérification comme par exemple des monoesters, diesters, triesters de sucrose et d'acide gras ou un mélange. L'ester de sucrose et d'acide gras peut se présenter sous forme d'un mélange d'esters à faible degré d'estérification comme par exemple un mélange de monoester et diester ou un mélange de monoester, diester et triester.

Dans le cas où l'on utilise un mélange d'esters de sucrose et d'acide gras, on préfère un mélange dans lequel les esters à faible degré d'estérification, en particulier les monoesters, sont majoritaires et représentent par exemple au

moins 50%, de préférence au moins 60% en poids du mélange d'esters de sucrose et d'acide gras.

On peut utiliser en particulier un mélange d'esters de sucrose et d'acides gras comprenant de 12 à 16 atomes de carbone, en particulier une mélange de mono, di et triesters d'acide laurique ou d'acide palmitique, ledit mélange pouvant comprendre de façon minoritaire (en une teneur inférieure ou égale à 40% en poids par rapport au poids du mélange d'esters de sucrose et d'acide gras) des esters de sucrose et d'acides gras dans lequel l'acide gras comprend plus de 16 atomes de carbone.

De préférence, l'ester de sucrose et d'acide gras utilisé dans la présente invention présente une HLB supérieure ou égale à 10, de préférence supérieure ou égale à 12.

Comme cela est bien connu, on entend par HLB (Hydrophilic-Lipophilic Balance) l'équilibre entre la dimension et la force du groupe hydrophile et la dimension et la force du groupe lipophile de l'agent tensioactif.

La valeur HLB selon GRIFFIN est définie dans J. Soc. Cosm. Chem. 1954 (volume 5), pages 249-256.

On peut citer à titre d'exemples d'esters ou de mélanges d'esters de sucrose et d'acide gras :

- le Surfhope SE COSME C-1416, présentant une HLB de 16, qui est un sucrose myristate comprenant environ 80% de monoester, le reste du mélange étant composé de di et triesters,
- le Surfhope SE COSME C-1216 dont le nom INCl est sucrose laurate, de HLB égale à 16 et comprend de 75 à 90% de monoester, le reste du mélange étant composé de di et triesters,
- le Surfhope SE COSME C-1215L dont le nom INCl est sucrose laurate, de HLB HLB égale à 15, comprenant environ 70% de monoesters, le reste du mélange étant composé de diesters et autres polyetsers,
- le Surfhope SE COSME C-1616, présentant une HLB de 16, qui est un mélange d'esters de saccharose et d'acides palmitique et/ou stéarique (nom INCI sucrose palmitate), comprenant de 75 à 90% de monoester, le reste du mélange étant composé de di et triesters, et pouvant comprendre du sucrose stéarate et sucrose palmitate stéarate.

On peut également citer l'ester portant le nom INCI sucrose laurate commercialisé par la société Dai-ichi Seiyaku sous la référence DK ester S-L18A, de HLB égale à 17, comprenant 70% de monoesters et 30% de di- et tri-esters.

On peut aussi citer à titre d'exemples d'esters ou de mélanges d'esters de sucrose d'acide gras :

- les produits vendus sous les dénominations F160, F140, F110, F90, F70, SL40 par la société CRODESTA, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di-, tri-, et tétra-ester, et le mono-laurate de sucrose;
- les produits vendus sous la dénomination RYOTO SUGAR ESTERS par exemple référencés B370 et correspondant au béhénate de saccharose formé de 20% de monoester et 80% de di-triester-polyester;
- le mono-di-palmito-stéarate de sucrose commercialisé par la société GOLDSCHMIDT sous la dénomination TEGOSOFT PSE.

Selon un mode de réalisation, on utilise le sucrose laurate.

Parmi les tensioactifs non ioniques selon l'invention, on peut également citer les saponines.

Les saponines peuvent être de préférence choisies parmi les saponines extraites d'arbres à savon (Sapindus mukurossi, Sapindus trifoliatus, sapindus saponaria), Réglisse (Glycyrrhiza glabra), marron d'Inde (Aesculus hippocastanum), bacoppa (Baccopa monneria), Salsepareille (Smilax medica, Smilax aspera, Smilax ornata), Bois de panama (Quillaja saponaria), saponaire (Saponaria officinalis), ginseng (Panax ginseng), yucca (Yucca schidigera), croix de malte (Tribulus terrestris), Juazirine (Zizyphus joazeiro), Jiaogulan (Gynostemma pentaphyllum), asperge d'Inde (Asparagus racemosus, luzerne (Medicago sativa) et leurs mélanges.

De préférence, le tensioactif non ionique est choisi dans le groupe constitué des alkylpolyglucosides, des alkylglycosides, des polyols, des esters de sucre, des alcools éthoxylés, les esters d'acide gras de sorbitane de polyoxyéthylène, et de leurs mélanges.

Selon un mode de réalisation, le tensioactif est un tensioactif non ionique choisi dans le groupe constitué du décyl glucoside, du coco-glucoside, du laurate de sucrose, du sorbitol, du monolaurate de sorbitane de polyoxyéthylène (Polysorbate 20), et de leurs mélanges.

Selon un mode de réalisation, le tensioactif est le décyl glucoside ou le coco-glucoside.

Selon un mode de réalisation, le tensioactif est un mélange de laurate de sucrose et de sorbitol.

Selon un autre mode de réalisation, le tensioactif est le monolaurate de sorbitane de polyoxyéthylène (Polysorbate 20).

Les compositions cosmétiques de l'invention peuvent comprendre un unique tensioactif tel que défini ci-dessus, ou un mélange de tensioactifs tels que définis ci-dessus.

En particulier, les compositions cosmétiques de l'invention comprennent un tensioactif.

Selon l'invention, la teneur total en tensioactif(s) (acide spiculisporique + tensioactif additionnel) dans la composition cosmétique de l'invention peut aller de 0,1% à 30% en masse par rapport à la masse totale de ladite composition.
De préférence, la teneur totale en tensioactif(s) (acide spiculisporique + tensioactif additionnel) selon l'invention va de 0,5% à 15%, et préférentiellement de 1% à 10% en masse par rapport à la masse totale de ladite composition.
Selon un mode de réalisation préféré, la teneur totale en tensioactif(s) (acide spiculisporique + tensioactif additionnel) selon l'invention est comprise entre 1% et 10%, de préférence entre 2% et 10%.

**Base**

Ainsi, les compositions selon l'invention, de préférence cosmétiques, dans lesquelles est compris l'acide spiculisporique, peuvent également comprendre une base organique ou minérale. La base, organique ou minérale, peut être une base de Bronsted-Lowry ou de Lewis.
Particulièrement, la (ou les) base(s) peut (ou peuvent) être choisie(s) parmi :

a) les alcanolamines, telles que les mono-, di- et triéthanolamines, l'isopropanolamine, le 2-amino-2-méthyl-1-propanol, ainsi que leurs dérivés,
b) les éthylènediamines oxyéthylénées et/ou oxypropylénées,
c) les hydroxydes minéraux ou organiques,
d) les silicates de métaux alcalins tels que les métasilicates de sodium,
e) les acides aminés de préférence basiques comme l'arginine, la lysine, l'ornithine, la citruline et l'histidine,
f) les carbonates et bicarbonates, particulièrement d'amine primaire, secondaire ou tertiaire, de métal alcalin ou alcalino-terreux, ou d'ammonium, et
g) les composés de formule (III) suivante :

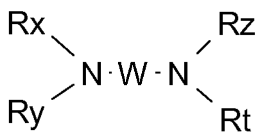
(III)

dans laquelle W est un reste alkylène en C1-C6 éventuellement substitué par un groupement hydroxyle ou un groupe alkyle en C1-C6 ; Rx, Ry, Rz et Rt, identiques ou différents, représentent un atome d'hydrogène, un groupe alkyle en C1-C6, hydroxyalkyle en C1-C6, ou aminoalkyle en C1-C6.
On peut citer, à titre d'exemple de tels composés de formule (III), le 1,3-diaminopropane, le 1,3-diamino-2-propanol, la spermine ou la spermidine.
Les hydroxydes minéraux ou organiques sont de préférence choisis parmi les hydroxydes d'un métal alcalin, les hydroxydes d'un métal alcalino-terreux, comme les hydroxydes de sodium ou de potassium, les hydroxydes d'un métal de transition, tels que les hydroxydes des métaux des groupes III, IV, V et VI de la classification périodique des éléments, les hydroxydes des lanthanides ou des actinides, les hydroxydes d'ammoniums quaternaires, et l'hydroxyde de guanidinium.
L'hydroxyde peut être formé in situ comme, par exemple, l'hydroxyde de guanidine, formé par réaction d'hydroxyde de calcium et de carbonate de guanidine.
Selon un mode de réalisation particulier de l'invention, la base peut être choisie dans le groupe des bases inorganiques comme l'hydroxyde de sodium, l'hydroxyde de potassium, l'hydroxyde de lithium, l'hydroxyde de césium, l'hydroxyde de calcium, l'hydroxyde de magnésium, l'hydroxyde de zinc, ou des bases semblables, d'un sel minéral basique et d'un sel organique basique contenant du lithium, du sodium, du potassium, du calcium, du magnésium, de l'ammonium, un acide aminé basique comme la lysine, l'arginine, l'histidine, l'ornithine, ou des semblables, un oligopeptide basique ayant ces acides aminés comme bases, des amines basiques telle que la monoéthanolamine, la diéthanolamine, la 2-(diméthylamino)éthanol, la triéthanolamine, triisopropanolamine, la diisopropanolamine, la monoisopropanolamine, l'ammoniaque, ou des bases semblables, d'autres bases organiques comme le carbonate de guanidine et autres bases semblables et de leurs mélanges.
Selon un mode de réalisation, la base est l'hydroxyde de potassium.
Selon un mode de réalisation, la base est l'arginine.
Dans le cadre de l'invention, et sauf mention contraire, la base utilisée est une base neutralisante, c'est-à-dire qu'elle permet de neutraliser l'acide spiculisporique pour former un sel dudit acide. On peut par exemple citer les sels de sodium, de potassium, de triéthanolamine et d'arginine de l'acide spiculisporique.
Selon un mode de réalisation, l'utilisation de deux moles d'acide spiculisporique permet de neutraliser deux fonctions carboxyliques dudit acide sans casser la fonction lactone. En particulier, deux moles de base sont utilisées

pour une mole d'acide spiculisporique dans les compositions cosmétiques susmentionnées.

Selon un mode de réalisation, l'utilisation de l'hydroxyde de potassium permet la formation du sel monopotassique, du sel dipotassique, ou du sel tripotassique de l'acide spiculisporique

La composition, de préférence cosmétique, déodorante peut également comprendre, outre l'acide spiculisporique tel que défini précédemment au moins un actif déodorant additionnel et /ou un actif anti-transpirant tels que définis ci-après

## Actifs déodorants

Les actifs déodorants peuvent être des agents bactériostatiques ou des agents bactéricides agissant sur les germes des odeurs axillaires, comme le 2,4,4'-trichloro-2'-hydroxydiphényléther (□Triclosan), le 2,4-dichloro-2'-hydroxydiphényléther, le 3',4',5'-trichlorosalicylanilide, la 1-(3',4'-dichlorophenyl)-3-(4'-chlorophenyl)urée (□Triclocarban) ou le 3,7,11-triméthyldodéca-2,5,10-triénol (□Farnesol) ; les sels d'ammonium quaternaires comme les sels de cetyltrimethylammonium, les sels de cétylpyridinium ; les polyols comme ceux de type glycérine, 1,3-propanediol (ZEMEA PROPANEDIOL® commercialisé par Dupont Tate and Lyle Bioproducts), le 1,2-décanediol (SYMCLARIOL® de la société Symrise) ; les dérivés de glycérine comme par exemple le Caprylic/Capric Glycerides (CAPMUL MCM® de Abitec), le Caprylate ou caprate de Glycerol (DERMOSOFT GMCY® et DERMOSOFT GMC® respectivement de STRAETMANS), le Polyglyceryl-2 Caprate (DERMOSOFT DGMC® de STRAETMANS) les dérivés de biguanide comme les sels de polyhexaméthylène biguanide ; la chlorhexidine et ses sels; le 4-Phenyl-4,4-dimethyl-2butanol (SYMDEO MPP® de Symrise) ; les cyclodextrines ; les chélatants tels que le Tetrasodium Glutamate Diacetate (CAS #51981-21-6) vendu sous le nom commercial DISSOLVINE GL-47-S® de Akzo Nobel, l'EDTA (acide Ethylendiamino Tétraacétique) et le DPTA (acide 1,3-diaminopropanetétraacétique).

Parmi les actifs déodorants conformes à l'invention, on peut aussi citer également

- les sels de zinc comme le salicylate de zinc, le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l'orthophosphate de zinc, le stéréate de zinc, le tartrate de zinc, l'acétate de zinc ou leurs mélanges ;
- des absorbeurs d'odeurs comme les zéolites notamment métalliques sans argent, les cyclodextrines, les silicates d'oxyde métallique telles que celles décrite dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- le bicarbonate de sodium ;
- l'acide salicylique et ses dérivés tels que l'acide n-octanoyl-5-salicylique
- l'alun.
- le triéthyl citrate

et leurs mélanges.

Les actifs déodorants additionnels peuvent être présents dans la composition selon l'invention à raison de 0,01 à 10 % en poids par rapport au poids total de la composition, et de préférence à raison de 0,1 à 5 % en poids.

## Actifs anti-transpirants

Parmi les actifs anti-transpirants, on peut citer les sels ou complexes anti-transpirants d'aluminium et/ou de zirconium, de préférence choisis parmi les halohydrates de d'aluminium ; les halohydrates d'aluminium et de zirconium, les complexes d'hydroxychlorure de zirconium et d'hydroxychlorure d'aluminium avec ou sans un acide aminé tels que ceux décrits dans le brevet US-3792068.

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate. Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les

complexes aluminium zirconium octachlorohydrex glycine, aluminium zirconium pentachlorohydrex glycine, aluminium zirconium tetrachlorohydrex glycine et aluminium zirconium trichlorohydrex glycine.

L'aluminium sesquichlorohydrate est notamment vendu sous la dénomination commercial REACH 301® par la société SUMMITREHEIS.

Parmi les complexes d'aluminium et de zirconium, on peut citer les complexes d'hydroxychlorure de zirconium et d'hydroxychlorure d'aluminium avec un acide aminé comme la glycine ayant pour nom INCI : ALUMINUM ZIRCONIUM TETRACHLOROHYDREX GLY par exemple celui commercialisé sous la dénomination REACH AZP-908-SUF® par la société SUMMITREHEIS.

On utilisera plus particulièrement le chlorhydrate d'aluminium sous forme activée ou non commercialisé sous les noms commerciaux LOCRON S FLA®, LOCRON P, LOCRON L.ZA par la société CLARIANT ; sous les dénominations commerciales MICRODRY ALUMINUM CHLOROHYDRATE®, MICRO-DRY 323®, CHLORHYDROL 50, REACH 103, REACH 501 par la société SUMMITREHEIS ; sous la dénomination commerciale WESTCHLOR 200® par la société WESTWOOD ; sous la dénomination commerciale ALOXICOLL PF 40® par la société GUILINI CHEMIE ; CLURON 50%® par la société INDUSTRIA QUIMICA DEL CENTRO ; CLOROHIDROXIDO ALUMINIO SO A 50%® par la société FINQUIMICA.

Comme autre actif anti-transpirant, on peut citer les particules de perlite expansée telles que celles obtenues par le procédé d'expansion décrit dans le brevet US 5,002,698.

Les perlites utilisables selon l'invention sont généralement des aluminosilicates d'origine volcanique et ont comme composition

70,0-75,0% en poids de silice SiO2
12,0-15,0% en poids d'oxyde d'aluminium oxyde Al2O3
3,0-5,0% d'oxyde de sodium Na2O
3,0-5,0% d'oxyde de potassium K2O
0,5-2% d'oxyde de fer Fe2O3 □
0,2-0,7% d'oxyde de magnésium MgO
0,5-1,5% d'oxyde de calcium CaO
0,05 - 0,15% d'oxyde de titane TiO2.

De préférence, les particules de perlite utilisées seront broyées ; elles sont dans ce cas dites Expanded Milled Perlite (EMP). Elles ont de préférence une taille de particule définie par un diamètre médian D50 allant de 0,5 à 50 μm et de préférence de 0,5 à 40 μm.

De préférence, les particules de perlite utilisées présentent une densité apparente non tassée à 25°C allant de 10 et 400 kg/m3 (Norme DIN 53468) et de préférence de 10 et 300 kg/m3.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 1 g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :

WET POINT : masse exprimée en grammes pour 100g de produit correspondant à l'obtention d'une pâte homogène lors de l'addition d'un solvant à une poudre.
FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant :

Protocole de mesure de l'absorption d'eau.

1) Matériel utilisé

Plaque de verre (25 x 25 mm)
Spatule (manche en bois et partie métallique (15 x 2,7mm)
Pinceau à poils de soie

Balance

2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

Les actifs anti-transpirants peuvent être présents dans la composition selon l'invention à raison de 0,001 à 30 % en poids par rapport au poids total de la composition et de préférence à raison de 0,5 à 25 % en poids.

## FORMES GALENIQUES

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Les compositions peuvent être notamment conditionnées sous forme pressurisée dans un dispositif aérosol ou dans un flacon pompe ; conditionnée dans un dispositif muni d'une paroi ajourée notamment une grille ; conditionnées dans un dispositif muni d'un applicateur à billes ("roll-on) ; conditionnées sous forme de bâtonnets (sticks), sous forme de poudre libre ou compactée. Elles contiennent à cet égard les ingrédients généralement utilisés dans ce type de produits et bien connus de l'homme de l'art.

.Dans un mode particulier l'invention concerne une composition telle que définie précédemment comprenant de l'acide spiculisporique sous forme libre, partiellement neutralisé ou totalement neutralisé par au moins une base minérale et/ou une base organique, caractérisée par le fait qu'elle se présente sous forme de gels aqueux ; de solution aqueuse ou hydroalcoolique, d'émulsion simple ou multiple, de dispersion vésiculaire de type ionique et/ou non ionique, de dispersion cire/phase aqueuse.

Selon une autre forme particulière de l'invention, les compositions selon l'invention peuvent être anhydres.

On entend par composition anhydre une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Selon une autre forme particulière de l'invention, les compositions selon l'invention peuvent être solides en particulier sous forme de bâtonnet ou stick.

Par «composition solide», on entend que la mesure de la force maximale mesurée en texturométrie lors de l'enfoncement d'une sonde dans l'échantillon de formule doit être au moins égale à 0,25 Newton, en particulier au moins égal à 0,30 Newton, notamment au moins égale 0,35 Newton, appréciée dans des conditions de mesure précises comme suit.

Les formules sont coulées à chaud dans des pots de 4 cm de diamètre et 3 cm de fond. Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant : une sonde de type bille en inox de diamètre 5 mm est amenée au contact de l'échantillon à une vitesse de 1 mm/s. Le système de mesure détecte l'interface avec l'échantillon avec un seuil de détection égal à 0,005 newtons. La sonde s'enfonce de 0,3 mm dans l'échantillon, à une vitesse de 0,1 mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

## Milieu physiologiquement acceptable

Une composition selon l'invention peut être une composition cosmétique, dermatologique ou pharmaceutique, de préférence cosmétique.

Une composition selon l'invention comprend notamment un milieu physiologiquement acceptable.

Dans le cadre de l'invention, et sauf mention contraire, on entend par "milieu physiologiquement acceptable", un milieu approprié aux applications cosmétiques, et convenant notamment à l'application d'une composition de l'invention sur la peau et/ou les cheveux. Le milieu physiologiquement acceptable est généralement adapté à la nature du support sur lequel doit être appliquée la composition, ainsi qu'à l'aspect sous lequel la composition doit être conditionnée.

## PHASE AQUEUSE

Les compositions selon l'invention destinées à l'usage cosmétique peuvent comporter au moins une phase aqueuse. Elles sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse des dites compositions contient de l'eau et en général d'autres solvants solubles ou miscibles dans l'eau. Les solvants solubles ou miscibles dans l'eau comprennent les mono alcools à chaîne courte par exemple en C1-C4 comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine, le propane 1,3 diol.

Selon l'invention, le pH de la composition selon l'invention peut être compris entre 3 et 10. De préférence, le pH est compris entre 5 et 8, et en particulier entre 5 et 6,5.

## EMULSIONNANTS

### Emulsionnants huile-dans-eau

Comme émulsionnants pouvant être utilisés dans les émulsions huile-dans-eau ou émulsions triples huile-dans-eau-dans-huile, on peut citer par exemple les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

On peut citer également les mélanges émulsionnants alcool gras/alkylpolyglycoside tels que sont décrits dans les demandes WO92/06778, WO95/13863 et WO98/47610 comme les produits commerciaux vendus par la société SEPPIC sous les appellation MONTANOV ®.

### Emulsionnants eau-dans-huile

Parmi les émulsionnants pouvant être utilisés dans les émulsions eau-dans-huile ou émulsions triples eau-dans-huile-dans-eau-dans-huile ou émulsions triples, on peut citer à titre d'exemple les alkyl dimethicone copolyols comme le Cetyl PEG/PPG-10/1 DIMETHICONE et plus particulièrement le mélange Cetyl PEG/PPG-10/1 DIMETICONE and Dimethicone (nom INCI) comme le produit vendu sous le nom commercial ABIL EM90 par la société GOLDSCHMIDT ou bien le mélange (Polyglyceryl-4-stearate and Cetyl PEG/PPG-10 (and) Dimethicone (and) Hexyl Laurate) comme le produit vendu sous le nom commercial ABIL WE09 par la même société.

Parmi les émulsionnants eau-dans-huile, on peut citer également les dimethicone copolyols comme le PEG-18/PPG-18 Dimethicone et plus particulièrement le mélange CYCLOPENTASILOXANE (and) PEG-18/PPG-18 Dimethicone (nom INCI) tel que le produit vendu par la société Dow Corning sous la dénomination commerciale Silicone DC 5225 C ou KF-6040 de la société Shin Etsu.

Parmi les émulsionnants eau-dans-huile, on peut citer également les émulsionnants non ioniques dérivés d'acide gras et de polyol, les alkylpolyglycosides (APG), les esters de sucres et leurs mélanges.

Comme émulsionnants non ioniques dérivés d'acide gras et de polyol, on peut utiliser notamment les esters d'acide gras et de polyol, l'acide gras ayant notamment une chaîne alkyle en C8-C24, et les polyols étant par exemple le glycérol et le sorbitan.

Comme esters d'acide gras et de polyol, on peut citer notamment les esters d'acide isostéarique et de polyols, les esters d'acide stéarique et de polyols, et leurs mélanges, en particulier les esters d'acide isostéarique et de glycérol et/ou de sorbitan.

Comme esters d'acide stéarique et de polyols, on peut citer notamment les esters de polyéthylèneglycol comme le PEG-30 Dipolyhydroxystearate tel que le produit commercialisé sous le nom Arlacel P135 par la société ICI.

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, , le mélange d'isostéarate de sorbitan et d'isostéarate de polyglycérol (3 moles) commercialisé sous la dénomination Arlacel 1690 par la société Unigema. et leurs mélanges.

L'émulsionnant peut être choisi aussi parmi les alkylpolyglycosides ayant un HLB inférieur à 7, par exemple ceux représentés par la formule générale (1) suivante :

R-O-(G)x (1)

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4, et G désigne notamment le glucose, le fructose ou le galactose.

Le radical alkyle insaturé peut comprendre une ou plusieurs insaturations éthyléniques, et en particulier une ou deux insaturations éthyléniques.

Comme alkylpolyglycosides de ce type, on peut citer les alkylpolyglucosides (G=glucose dans la formule (I)), et notamment les composés de formule (I) dans laquelle R représente plus particulièrement un radical oléyle (radical insaturé en C18) ou isostéaryle (radical saturé en C18), G désigne le glucose, x est une valeur allant de 1 à 2, notamment l'isostéaryl-glucoside, l'oléyl-glucoside et leurs mélanges. Cet alkylpolyglucoside peut être utilisé en mélange avec un co-émulsionnant, plus spécialement avec un alcool gras et notamment un alcool gras ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleyl-glucoside, éventuellement sous forme d'une composition autoémulsionnante, comme décrit par exemple dans le document WO-A-92/06778. On peut utiliser par exemple le mélange d'isostéaryl-glucoside et d'alcool isostéarylique, commercialisé sous la dénomination Montanov WO 18 par la société SEPPIC ainsi que le mélange octyldodécanol et octyldodecylxyloside commercialisé sous la dénomination FLUDANOV 20X par la société SEPPIC.

On peut également citer les polyoléfines à terminaison succinique, comme les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol ou le produit commercial CHEMCINNATE 2000.

La quantité totale en émulsionnants dans la composition sera de préférence dans la composition selon l'invention à des teneurs en matière active allant de 1 à 8% en poids et plus particulièrement de 2 à 6% en poids par rapport au poids total de la composition.

## PHASE GRASSE

Les compositions selon l'invention peuvent contenir au moins une phase liquide organique non-miscible dans l'eau appelée phase grasse. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase liquide organique non-miscible dans l'eau conforme à l'invention comprend généralement au moins une huile volatile et/ou une huile non volatile et éventuellement au moins un agent structurant.

Par « huile », on entend un corps gras liquide à température ambiante (25 °C) et pression atmosphérique (760mm de Hg soit 105 Pa). L'huile peut être volatile ou non volatile.

Par « huile volatile », on entend au sens de l'invention une huile susceptible de s'évaporer au contact de la peau ou de la fibre kératinique en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur la peau ou la fibre kératinique à température ambiante et pression atmosphérique au moins plusieurs heures et ayant notamment une pression de vapeur inférieure à 10-3 mm de Hg (0,13 Pa).

L'huile peut être choisie parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges.

Plus précisément, par « huile hydrocarbonée », on entend une huile comportant principalement des atomes

de carbone et d'hydrogène et éventuellement une ou plusieurs fonctions choisies parmi les fonctions hydroxyle, ester, éther, carboxylique. Généralement, l'huile présente une viscosité de 0,5 à 100 000 mPa.s, de préférence de 50 à 50 000 mPa.s et de préférence encore de 100 à 300 000 mPa.s.

A titre d'exemple d'huile volatile utilisable dans l'invention, on peut citer :

- les huiles hydrocarbonées volatiles choisies parmi les huiles hydrocarbonées ayant de 8 à 16 atomes de carbones, et notamment les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en $C_8$-$C_{16}$, le néopentanoate d'isohexyle, et leurs mélanges. D'autres huiles hydrocarbonées volatiles comme les distillats de pétrole, notamment ceux vendus sous la dénomination Shell Soit par la société SHELL, peuvent aussi être utilisées ; les alcanes linéaires volatils comme ceux décrits dans la demande de brevet de la société Cognis DE10 2008 012 457.
- les silicones volatiles, comme par exemple les huiles de silicones linéaires ou cycliques volatiles, notamment celles ayant une viscosité ≤ 8 centistokes (8 10-6 $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane
- et leurs mélanges.

On peut également citer les huiles linéaires alkyltrisiloxanes volatiles de formule générale (I) :

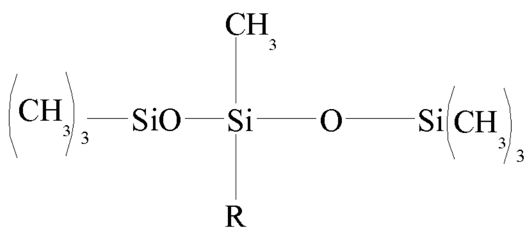

où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

A titre d'exemple d'huile non volatile utilisable dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles les huiles de germe de blé, d'olive, l'huile d'amande douce, de palme, de colza, de coton, de luzerne, de pavot, de potimarron, de courge, de cassis, d'onagre, de millet, d'orge, de quinoa, de seigle, de carthame, de bancoulier, de passiflore, de rosier muscat, de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone ;
- les esters de synthèse notamment d'acides gras comme les huiles de formule $R_1COOR_2$ dans laquelle $R_1$ représente le reste d'un acide gras supérieur linéaire ou ramifié comportant de 1 à 40 atomes de carbone et R2 représente une chaîne hydrocarbonée notamment ramifiée contenant de 1 à 40 atomes de carbone avec $R_1 + R_2 \geq 10$ comme

par exemple l'huile de Purcellin (octanoate de cétostéaryle), l'isononanoate d'isononyle, le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les octanoates, décanoates ou ricinoléates d'alcools ou de polyalcools comme le dioctanoate de propylène glycol ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;

- des alcools gras liquides à température ambiante à chaîne carbonée ramifiée et/ou insaturée ayant de 12 à 26 atomes de carbone comme l'octyl dodécanol, l'alcool isostéarylique, le 2-butyloctanol, le 2-hexyl décanol, le 2-undécyl pentadécanol, l'alcool oléique ;
- les acides gras supérieurs tels que l'acide oléique, l'acide linoléique, l'acide linolénique ;
- les carbonates ;
- les acétates ;
- les citrates ;
- les huiles fluorées éventuellement partiellement hydrocarbonées et/ou siliconées comme les huiles fluorosiliconées, les polyéthers fluorés, les silicones fluorées telles que décrit dans le document EP-A-847752;
- les huiles siliconées comme les polydiméthylsiloxanes (PDMS) non volatiles, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et
- leurs mélanges.

## Additifs

Les compositions cosmétiques selon l'invention peuvent comprendre en outre des adjuvants cosmétiques choisis parmi les adoucissants, les antioxydants, les opacifiants, les stabilisants, les agents hydratants, les vitamines, des bactéricides, les conservateurs, les polymères, les parfums, un agent structurant de phase phase grasse en particulier choisi parmi les cires, les composés pâteux, les gélifiants lipophiles minéraux ou organiques ; les charges organiques ou inorganiques ; les agents épaississants ou de mise en suspension, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

### Cire(s)

La cire est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, Cire de Tournesol raffinée commercialisée sous la dénomination SUNFLOWER WAX par KOSTER KEUNEN, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C8-C32. Parmi celles-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone (C30-45 ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydroxystéaryloxy)stéarate d'alkyle en C20-C40 (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme micro-cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS, les produits commerciaux PERFOMALEN 400 POLYETHYLENE et PERFORMALENE 500-L POLYETHYLENE de NEW PHASE TECHNOLOGIES, le PERFORMALENE 655 POLYETHYLENE ou les cires de paraffine comme la cire ayant pour nom INCI, MICROCRISTALLINE WAX and SYNTHETIC WAX et vendue sous le nom commercial MICROLEASE par la Société SOCHIBO. ; les micro cires de polytétrafluoroéthylène telles que celles commercialisées sous les dénominations de Microslip 519® et 519 L® par la société MICRO POWDERS.

Composés pâteux

Par « composé pâteux » au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

Le composé pâteux est de préférence choisi parmi les composés synthétiques et les composés d'origine végétale. Un composé pâteux peut être obtenu par synthèse à partir de produits de départ d'origine végétale.

Le composé pâteux peut avantageusement être choisi parmi :

- la lanoline et ses dérivés,
- les composés siliconés polymères ou non,
- les composés fluorés polymères ou non,
- les polymères vinyliques, notamment :
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en $C_8$-$C_{30}$,
- les oligomères homo et copolymères d'esters vinyliques ayant des groupements alkyles en $C_8$-$C_{30}$, et
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,

- les esters,
- leurs mélanges.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ®, et Risocast DA-L ®,
- les esters de dimère diol et dimère diacide, le cas échéant, estérifiés sur leur(s) fonction(s) alcool(s) ou acide(s) libre(s) par des radicaux acides ou alcools tels que le Plandool-G,
- leurs mélanges.

Gélifiants lipophiles minéraux

Comme gélifiant lipophile minéral, on peut citer les argiles éventuellement modifiées comme les hectorites modifiées par un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT, des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

Gélifiants lipophiles organiques

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton®

par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Epaississants et agents de suspension

Les épaississants peuvent être choisis parmi les les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose, la cétylhydroxyéthylcellulose ; les polysaccharides et notamment les gommes telles que la gomme de Xanthane, les gommes de guar hydroxypropylée ; les silices comme par exemple la Bentone Gel MIO vendue par la société NL INDUSTRIES ou la Veegum Ultra, vendue par la société POLYPLASTIC.

Les épaississants peuvent également être cationiques comme par exemple le POLYQUATERNIUM-37 commercialisé sous la dénomination Salcare SC95 (Polyquaternium-37 (And) Minerai Oil (And) PPG-1 Trideceth-6) ou Salcare SC96 (Polyquaternium-37 (And) Propylene Glycol Dicaprylate/Dicaprate (And) PPG-1-Trideceth-6) ou d'autre polymère cationiques réticulés comme par exemple ceux de nom CTFA Copolymère Ethylacrylate / Dimethylamino Ethyl Methacrylate Cationique En Emulsion.

**Poudre organique**

Selon une forme particulière de l'invention, les compositions selon l'invention contiendront en plus une poudre organique.

On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les fibres de nylon 6,6 notamment les fibres de polyamide commercialisées par les Etablissements P Bonte sous le nom Polyamide 0.9 Dtex 0.3 mm (non INCI : Nylon 6,6 ou Polyamide-6,6) ayant un diamètre moyen de 6 $\mu$m, un poids d'environ 0,9 dtex et une longueur allant de 0,3 mm à 1,5 mm ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les microsphères de poly methacrylate de methyle creuses (granulometrie : 6,5 - 10,5 $\mu$) commercialisées sous la dénomination GANZPEARL GMP 0800 par Ganz Chemical; micro-billes de copolymere methacrylate de methyle/dimethacrylate d'ethylene glycol (taille: 6.5-10.5 $\mu$) commercialisées sous la dénomination GANZPEARL GMP 0820 par Ganz Chemical ou MICROSPONGE 5640 par la société Amcol Health & Beauty Solutions; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 $\mu$m et masse volumique 40 kg/$m^3$), 551 DE 20 (granulométrie d'environ 30 $\mu$m et masse volumique 65 kg/$m^3$), 551 DE 50 (granulométrie d'environ 40 $\mu$m), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société

National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 μm et notamment allant de 0,02 μm à 1 μm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

Agents de suspension

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

D'autres agents de suspension peuvent être utilisés, en l'occurrence dans des milieux hydrophiles (aqueux et/ou éthanoliques). Il s'agit de dérivés cellulosiques, de xanthane, guar amidon, caroube, agar agar.

Les agents de suspension sont présents de préférence dans des quantités allant de 0,1 à 5% en poids et plus préférentiellement de 0,2 à 2% en poids par rapport au poids total de la composition.

Les quantités de ces différents constituants pouvant être présents dans la composition cosmétique selon l'invention sont celles classiquement utilisées dans compositions pour le traitement de la transpiration.

**Aérosols**

Les compositions selon l'invention peuvent encore être pressurisées et être conditionnées dans un dispositif aérosol constitué par :

(A) un récipient comprenant une composition anti-transpirante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

Les compositions contenant les particules de perlite telles que définies précédemment et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

Les expressions « compris entre ... et ... » et « allant de ... à ... », ou « au moins de.. » doivent se comprendre bornes incluses, sauf si le contraire est spécifié. Les exemples et figures qui suivent sont présentés à titre illustratif et non limitatif de l'invention. Les composés sont, selon le cas, cités en noms chimiques ou en noms CTFA (International Cosmetic Ingredient Dictionary and Handbook).

Dans ces exemples, les quantités des ingrédients des compositions sont données en % en poids par rapport au poids total de la composition.

**Exemple 1 : compositions A et B selon l'invention**

Deux compositions A et B selon l'invention comprenant de l'acide spiculisporique ont été préparées selon un procédé classique pour l'homme du métier, tel que celui ci-dessous ;

- Dispersion de l'acide spiculisporique dans l'eau sous agitation à température ambiante
- Ajout progressif sous agitation de la base pour avoir le ratio molaire

R1 nmole base /nmoles S-acide =1,1

La masse de base à utiliser pour neutraliser l'acide spiculisporique (acide-S) est définie de la façon suivante

$$masse_{(base)}g = 2 \times \frac{masse_{(acide-S)}g}{MM_{(acide-S)}g.mol^{-1}} \times MM_{(base)}g.mol^{-1}$$

Formules brutes et masses moléculaires de l'acide spiculisporique et de différentes bases utilisables selon l'invention :

| | Formule brute | Masse molaire (g.mol$^{-1}$) |
|---|---|---|
| Acide spiculisporique | $C_{17}H_{28}O_6$ | 328,4 |
| Hydroxyde de sodium | NaOH | 40 |
| Hydroxyde de potassium | KOH | 56,1 |
| Triétanolamine (TEA) | $C_6H_{15}NO_3$ | 149,19 |
| L-arginine | $C_6H_{14}N_4O_2$ | 174,2 |

Composition des formules préparées :

| Ingrédients | Composition A : % des ingrédients | Composition B : % des ingrédients |
|---|---|---|
| Arginine (Disponible sous la dénomination L-Arginine® commercialisé par la société Ajinomoto) | Ratio $R_1$=1,1 5,84 | Ratio $R_1$=1,1 2,92 |
| Acide spiculisporique (disponible sous la dénomination SPICULISPORIC ACID® commercialisé par la société IWATA CHEMICAL) | 10 | 5 |
| EAU | 84,16 | 92,08 |

**Exemple 2 : Mise en évidence de l'activité bactéricide vis-à-vis du germe Cornebacterium xerosis d'une composition cosmétique contenant de l'acide spiculisporique**

**PRINCIPE**

Détermination quantitative de l'activité d'une formule cosmétique vis-à-vis de Corynebacterium xerosis, micro-organisme impliqué dans les phénomènes liés à l'odeur axillaire. Ce micro-organisme est mis en conditions optimales de croissance.

**PROTOCOLE**

- La souche modèle utilisée est : Corynebacterium xerosis, Collection de l'Institut Pasteur CIP 5216 (bactérie)

La souche est mise en contact avec la formule à tester dans un milieu de culture liquide adapté dans les rapports suivants :

- 10% de l'inoculum microbien à $10^8$ germes/ml
- 10% de la formule à tester,
- 80% de milieu de culture liquide (bouillon Tryptocaséine soja)

En parallèle, un témoin de croissance, dans lequel la formule à tester est remplacée par du diluant (tryptone sel), est préparé dans les mêmes conditions.
Les échantillons sont placés dans incubateur rotatif à 35°C et maintenus sous agitation pendant toute la durée du test.
Aprè 2, 6, et 24 heures de contact, le nombre de micro-organismes vivants restant dans le mélange est évalué.
Les résultats sont exprimés en logarithme du nombre de micro-organismes par millilitre de mélange.

**COMPOSITIONS**

- Compositions A et B selon l'invention comprenant de l'acide spiculisporique, et composition témoin testées :;

  Composition A : solution aqueuse à 10% d'acide spiculisporique neutralisé par une base selon un ratio Rbase/RS-acid= 1,1
  Composition B : solution aqueuse à 5% d'acide spiculisporique neutralisée par une base selon un ratio Rbase/RS-acid= 1,1

**RESULTATS**

24 h après inoculation avec 10% d'un inoculum microbien à 10^8 germes/ml une décontamination de 6,6log et 4,9log par rapport au témoin de croissance a été obtenue respectivement avec les compositions A et B.
L'évolution du nombre de micro-organismes par millilitre d'échantillon (en Log) est présentée dans les tableaux ci-après :

| *C. xerosis (log)* à | t0 | t2h | t6h | t24h | différence *C. xerosis (log)* après 24H de temps de contact par rapport au témoin |
|---|---|---|---|---|---|
| Composition A | 7,1 | <1,3 | <1,3 | 1,3 | <-6,6 |
| témoin | 7,1 | 7,1 | 7,0 | 7,9 | |

Par rapport aux témoins de croissance, la composition A présente une excellente activité sur C. *xerosis* dès 2h de contact (plus de 5 Log de réduction).

| *C. xerosis (log)* à | t0 | t2h | t6h | t24h | différence *C. xerosis (log)* après 24H de temps de contact par rapport au témoin |
|---|---|---|---|---|---|
| Composition B | 7,1 | 4,4 | 3,5 | 3,0 | -4,9 |
| témoin | 7,1 | 7,1 | 7,0 | 7,9 | |

Par rapport aux témoins de croissance, la composition B présente une excellente activité antimicrobienne sur C. xerosis (réduction de 5 Log à 24h)
L'acide spiculisporique sous forme libre, partiellement ou totalement neutralisé par au moins une base minérale et/ou organique peut donc être mise en oeuvre dans une composition permettant ainsi d'inhiber la croissance de *Corynebacterium xerosis,* utile pour le traitement des mauvaises odeurs produites par la décomposition de la sueur.

**Exemple 2 : Formule déodorante : Roll-on** (% massique par rapport à la masse de la composition totale)

| | |
|---|---|
| PARFUM | 0,7 |
| GOMME DE XANTHANE | 0,6 |
| PROPANEDIOL | 5 |
| ALCOOL 96% ET EAU | 45 |
| Acide Spiculisporique | 5 |
| ARIGININE | 2,92 |
| GLYCERYL CAPRYLATE | 1,5 |
| EAU | QSP 100 |

La composition produit un effet déodorant.

**Exemple 3 : Formule déodorante : Roll-on** (% massique par rapport à la masse de la composition totale)

| | |
|---|---|
| PARFUM | 1 |
| GOMME DE XANTHANE | 0,4 |
| PECTINE | 2 |
| MELANGE DE :<br>- TRIGLYCERIDES D'ACIDES CAPRYLIQUE/CAPRIQUE,<br>- HUILE DE JOJOBA, ET<br>- OXYDE DE DIOCTYLE | 20 |
| Acide Spiculisporique | 5 |
| ARIGININE | 3 |
| CARBONATE DE CALCIUM | 0.05 |
| CONSERVATEUR | 0.5 |
| EAU | QSP 100 |

La composition produit un effet déodorant.

## Revendications

1. Utilisation cosmétique comme actif déodorant de l'acide spiculisporique sous forme libre, partiellement neutralisé ou totalement neutralisé par au moins une base minérale et/ou une base organique.

2. Utilisation selon la revendication 1, dans laquelle l'acide spiculisporique est contenu dans une composition comprenant un milieu physiologiquement acceptable, et plus particulièrement comprenant en outre une phase aqueuse.

3. Utilisation selon l'une quelconque des revendications 1 et 2, dans laquelle l'acide spiculisporique est partiellement neutralisé ou totalement neutralisé par au moins une base organique.

4. Utilisation selon l'une quelconque des revendications 1 à 3, dans laquelle la base organique est choisie dans le groupe constitué des acides aminés basiques, des oligopeptides basiques, des amines basiques et de leurs mélanges.

**5.** Utilisation selon l'une quelconque des revendications 1 à 4, dans laquelle la base organique est choisie dans le groupe constitué de l'arginine, de la triéthanolamine, et de leurs mélanges, de préférence est l'arginine.

**6.** Utilisation selon l'une quelconque des revendications 1 à 5, dans laquelle le ratio $R_1$ du nombre de moles de base sur le nombre de moles d'acide spiculisporique est strictement supérieur à 1.0.

**7.** Utilisation selon l'une quelconque des revendications 1 à 6, dans laquelle le ratio $R_1$ du nombre de moles de base sur le nombre de moles d'acide spiculisporique est strictement supérieur à 1.0 et inférieur ou égal à 2.50.

**8.** Utilisation selon l'une quelconque des revendications 1 à 7, dans laquelle le ratio $R_1$ du nombre de moles de base sur le nombre de moles d'acide spiculisporique est supérieur ou égal à 1.10 et inférieur ou égal à 2.0.

**9.** Utilisation selon l'une quelconque des revendications 2 à 8, dans laquelle l'acide spiculisporique est présent dans la composition dans une teneur comprise entre 0.1% et 15%, en particulier entre 0.5% et 10%, et encore plus particulièrement entre 1% et 10% en poids par rapport au poids total de la composition.

**10.** Utilisation selon l'une quelconque des revendications 2 à 9, dans laquelle l'acide spiculisporique est contenu dans une composition comprenant au moins un tensioactif.

**11.** Composition **caractérisée par le fait qu'**elle comprend dans un milieu physiologiquement acceptable:

a) au moins un acide spiculisporique tel que défini dans l'une quelconque des revendications précédentes et
b) au moins un actif déodorant additionnel et/ou au moins un actif anti-transpirant.

**12.** Composition selon la revendication 11, conditionnée :

(i) sous forme pressurisée dans un dispositif aérosol ou dans un flacon pompe ;
(ii) dans un dispositif muni d'une paroi ajourée notamment une grille ;
(iii) dans un dispositif muni d'un applicateur à bille (« roll-on) ;
(iv) sous forme de bâtonnet (stick) ; ou
(v) sous forme de poudre libre ou compactée.

**13.** Composition selon l'une quelconque des revendications 11 et 12, **caractérisée par le fait qu'**elle se présente sous forme de gels aqueux ; de solution aqueuse ou hydroalcoolique, d'émulsion simple ou multiple, de dispersion vésiculaire de type ionique et/ou non ionique, de dispersion cire/phase aqueuse.

**14.** Composition selon l'une quelconque des revendications 11 à 13 ; **caractérisée par le fait qu'**elle est anhydre.

**15.** Procédé cosmétique pour traiter les odeurs corporelles humaines, en particulier des aisselles ou des pieds, consistant à appliquer sur les matières kératiniques humaines au moins un acide spiculisporique selon l'une quelconque des revendications 1 à 10 ou une composition le contenant dans un milieu physiologiquement acceptable, selon l'une quelconque des revendications 2 à 10.

## Patentansprüche

**1.** Kosmetische Verwendung von Spiculisporsäure in freier oder teilweise oder vollständig durch mindestens eine anorganische Base und/oder eine organische Base neutralisierter Form als desodorierender Wirkstoff.

**2.** Verwendung nach Anspruch 1, wobei die Spiculisporsäure in einer Zusammensetzung, die ein physiologisch unbedenkliches Medium umfasst und spezieller außerdem eine wässrige Phase umfasst, enthalten ist.

**3.** Verwendung nach einem der Ansprüche 1 und 2, wobei die Spiculisporsäure teilweise oder vollständig durch mindestens eine organische Base neutralisiert ist.

**4.** Verwendung nach einem der Ansprüche 1 bis 3, wobei die organische Base aus der Gruppe bestehend aus basischen Aminosäuren, basischen Oligopeptiden, basischen Aminen und Mischungen davon ausgewählt ist.

5. Verwendung nach einem der Ansprüche 1 bis 4, wobei die organische Base aus der Gruppe bestehend aus Arginin, Triethanolamin und Mischungen davon ausgewählt ist und vorzugsweise Arginin ist.

6. Verwendung nach einem der Ansprüche 1 bis 5, wobei das Verhältnis $R_1$ der Zahl der Mole der Base zur Zahl der Mole der Spiculisporsäure streng größer als 1,0 ist.

7. Verwendung nach einem der Ansprüche 1 bis 6, wobei das Verhältnis $R_1$ der Zahl der Mole der Base zur Zahl der Mole der Spiculisporsäure streng größer als 1,0 und kleiner oder gleich 2,50 ist.

8. Verwendung nach einem der Ansprüche 1 bis 7, wobei das Verhältnis $R_1$ der Zahl der Mole der Base zur Zahl der Mole der Spiculisporsäure größer oder gleich 1,10 und kleiner oder gleich 2,0 ist.

9. Verwendung nach einem der Ansprüche 2 bis 8, wobei die Spiculisporsäure in der Zusammensetzung in einem Gehalt zwischen 0,1 und 15 Gew.-%, insbesondere zwischen 0,5 und 10 Gew.-% und noch spezieller zwischen 1 und 10 Gew.-%, bezogen auf das Gesamtgewicht der Zusammensetzung, vorliegt.

10. Verwendung nach einem der Ansprüche 2 bis 9, wobei die Spiculisporsäure in einer Zusammensetzung, die mindestens ein Tensid umfasst, enthalten ist.

11. Zusammensetzung, **dadurch gekennzeichnet, dass** sie in einem physiologisch unbedenklichen Medium Folgendes enthält:

    a) mindestens eine Spiculisporsäure wie in einem der vorhergehenden Ansprüche definiert und
    b) mindestens einen zusätzlichen desodorierenden Wirkstoff und/oder mindestens einen schweißhemmenden Wirkstoff.

12. Zusammensetzung nach Anspruch 11, die

    (i) druckbeaufschlagt in einer Aerosolvorrichtung oder in einer Pumpflasche;
    (ii) in einer Vorrichtung mit einer durchbrochenen Wand, insbesondere einem Gitter;
    (iii) in einer Vorrichtung mit einem Kugelapplikator ("Roller");
    (iv) in Form eines Stifts oder
    (v) in Form eines losen oder kompaktierten Puders

    konfektioniert ist.

13. Zusammensetzung nach einem der Ansprüche 11 und 12, **dadurch gekennzeichnet, dass** sie in Form eines wässrigen Gels; einer wässrigen oder wässrigalkoholischen Lösung, einer einfachen oder multiplen Emulsion, einer Vesikeldispersion vom ionischen und/oder nichtionischen Typ oder einer Dispersion von Wachs und wässriger Phase vorliegt.

14. Zusammensetzung nach einem der Ansprüche 11 bis 13, **dadurch gekennzeichnet, dass** sie wasserfrei ist.

15. Kosmetisches Verfahren zur Behandlung von menschlichen Körpergerüchen, insbesondere der Achseln oder der Füße, das darin besteht, dass man auf menschlichen Keratinmaterialien mindestens eine Spiculisporsäure nach einem der Ansprüche 1 bis 10 oder eine diese in einem physiologisch unbedenklichen Medium enthaltende Zusammensetzung nach einem der Ansprüche 2 bis 10 aufbringt.

## Claims

1. Cosmetic use, as a deodorant active agent, of spiculisporic acid in free form or in a form partially neutralized or totally neutralized with at least one mineral base and/or one organic base.

2. Use according to Claim 1, in which the spiculisporic acid is contained in a composition comprising a physiologically acceptable medium, and more particularly also comprising an aqueous phase.

3. Use according to either of Claims 1 and 2, in which the spiculisporic acid is partially neutralized or totally neutralized

with at least one organic base.

4. Use according to any one of Claims 1 to 3, in which the organic base is chosen from the group formed from basic amino acids, basic oligopeptides and basic amines, and mixtures thereof.

5. Use according to any one of Claims 1 to 4, in which the organic base is chosen from the group formed from arginine and triethanolamine, and mixtures thereof, and is preferably arginine.

6. Use according to any one of Claims 1 to 5, in which the ratio $R_1$ of the number of moles of base to the number of moles of spiculisporic acid is strictly greater than 1.0.

7. Use according to any one of Claims 1 to 6, in which the ratio $R_1$ of the number of moles of base to the number of moles of spiculisporic acid is strictly greater than 1.0 and less than or equal to 2.50.

8. Use according to any one of Claims 1 to 7, in which the ratio $R_1$ of the number of moles of base to the number of moles of spiculisporic acid is greater than or equal to 1.10 and less than or equal to 2.0.

9. Use according to any one of Claims 2 to 8, in which the spiculisporic acid is present in the composition in a content of between 0.1% and 15% by weight, in particular between 0.5% and 10% by weight and even more particularly between 1% and 10% by weight relative to the total weight of the composition.

10. Use according to any one of Claims 2 to 9, in which the spiculisporic acid is contained in a composition comprising at least one surfactant.

11. Composition, **characterized in that** it comprises, in a physiologically acceptable medium:

a) at least one spiculisporic acid as defined in any one of the preceding claims, and
b) at least one additional deodorant active agent and/or at least one antiperspirant active agent.

12. Composition according to Claim 11, conditioned:

(i) in pressurized form in an aerosol device or in a pump-action bottle;
(ii) in a device equipped with an openwork wall, especially a grate;
(iii) in a device equipped with a ball applicator ("roll-on") ;
(iv) in the form of a wand (stick); or
(v) in the form of a loose or compacted powder.

13. Composition according to either of Claims 11 and 12, **characterized in that** it is in the form of an aqueous gel; an aqueous or aqueous-alcoholic solution, a simple or multiple emulsion, a vesicular dispersion of ionic and/or nonionic type, or a wax/aqueous phase dispersion.

14. Composition according to any one of Claims 11 to 13, **characterized in that** it is anhydrous.

15. Cosmetic process for treating human body odors, in particular of the armpits or feet, which consists in applying to the human keratin materials at least one spiculisporic acid according to any one of Claims 1 to 10 or a composition containing same in a physiologically acceptable medium, according to any one of Claims 2 to 10.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2005063928 A
- US 2005084464 A
- US 2005084474 A
- EP 1658863 A
- US 6916465 B
- US 3792068 A
- US 5002698 A
- WO 9206778 A
- WO 9513863 A
- WO 9847610 A
- DE 102008012457
- EP 847752 A
- FR 2792190 A
- US 5874069 A
- US 5919441 A
- US 6051216 A
- US 5981680 A


### Littérature non-brevet citée dans la description


- **GRIFFIN.** *J. Soc. Cosm. Chem.,* 1954, vol. 5, 249-256
- **C. FOX.** *Cosmetics and Toiletries,* Novembre 1986, vol. 101, 101-112